# EUROPEAN PATENT SPECIFICATION

(11) **EP 1 405 856 B1**
(45) Date of publication and mention of the grant of the patent: **26.09.2018**
(21) Application number: 02733463.0
(22) Date of filing: 11.06.2002
(51) Int. Cl.: C07H 5/06

(54) **CRYSTALS OF OLIGOSACCHARIDES AND PROCESSES FOR PREPARATION THEREOF**
KRISTALLE VON OLIGOSACCHARIDEN UND VERFAHREN ZU DEREN HERSTELLUNG
CRISTAUX D'OLIGOSACCHARIDES ET PROCEDES DE PREPARATION CORRESPONDANTS

(30) Priority: 11.06.2001 JP 2001175930
(43) Date of publication of application: 07.04.2004
(73) Proprietor: Kyowa Hakko Bio Co., Ltd., Chiyoda-ku Tokyo 100-8185 (JP)
(72) Inventor: SHIMOSE, Tsuyoshi, Technical Research Laboratories, Hofu-shi, Yamaguchi 747-8522 (JP); NAGANO, Hiroshi, Technical Research Laboratories, Hofu-shi, Yamaguchi 747-8522 (JP); ARIMOTO, Masaru, Osaka Branch Office, Osaka-shi, Osaka 530-8280 (JP); MURATA, Hideki, Technical Research Laboratories, Hofu-shi, Yamaguchi 747-8522 (JP)
(74) Representative: D Young & Co LLP
(86) International application number: PCT/JP2002/005787
(87) International publication number: WO 2002/100875

(56) References cited:
- EP-A1- 0 474 046
- EP-A1- 0 670 327
- JP-A- 7 082 287
- JP-A- 60 232 095
- JP-A- 63 246 391
- JP-A- 2000 279 191
- JP-B2- 57 005 239
- KUHN R ET AL: "KRISTALLISATION UND KONSTITUTIONSERMITTLUNG DER LACTO-N-FUCOPENTAOSE" CHEMISCHE BERICHTE, VERLAG CHEMIE GMBH. WEINHEIM, DE, vol. 89, no. 11, 1996, pages 2514-2523, XP009071510 ISSN: 0009-2940
- RICHARD KUHN ET AL: "KRISTALLISIERTE FUCOSIDO-LACTOSE" CHEMISCHE BERICHTE, VERLAG CHEMIE GMBH. WEINHEIM, DE, vol. 89, no. 11, November 1956 (1956-11), page 2513, XP009074773 ISSN: 0009-2940
- IMBERTY A ET AL: "CRYSTAL STRUCTURE AND CONFORMATIONAL FEATURES OF .ALPHA.-PANOSE" CARBOHYDRATE RESEARCH, ELSEVIER SCIENTIFIC PUBLISHING COMPANY. AMSTERDAM, NL, vol. 181, no. 1, 1 October 1988 (1988-10-01), pages 41-55, XP009071514 ISSN: 0008-6215
- TOKURA SODA: 'Tatorui kagaku', 15 December 1955, KYORITSU SHUPPAN CO., LTD. XP002958980 * page 13,202 - page 203 *
- ACHER, A.J. ET AL.: 'Studies in the ganglioside series. V. Synthesis of 2-acetamido-2-deoxy-O-beta-D-glucopyranosyl -(1 3)-O-beta-D-galactopyranosyl-(1 4)-D-glucose' J. ORG. CHEM. vol. 35, no. 7, 1970, pages 2436 - 2437, XP002958981
- PEREZ, S. ET AL.: 'Crystal and molecular structure of a histo-blood group antigen involved in cell adhesion: the Lewis X trisaccharide' GLYCOBIOLOGY vol. 6, no. 5, 1996, pages 537 - 542, XP002958982
- SAUTER, N.K. ET AL.: 'Binding of influenza virus hemagg lutinin to analogs of its cell-surface receptor, sialic acid: analysis by proton nuclear magnetic resonance spectroscopy and X-ray crystallography' BIOCHEMISTRY vol. 31, no. 40, 1992, pages 9609 - 9621, XP002958983
- PAULSEN, H. ET AL.: 'Regioselektive glycosylierung an stell ungen 3' und 4' von lactose-derivaten' CARBOHYDRATE RESEARCH vol. 169, 1987, pages 105 - 125, XP002958984
- HERMANSSON, K. ET AL.: 'A 1H and 13C NRM study of oligosaccharides from human milk. Application of the computer program CASPER' CARBOHYDRATE RESEARCH vol. 235, 1992, pages 69 - 81, XP002958985
- SARKAR A K ET AL: "2,3,4,6-Tetra-O-benzyl-beta-D-galactopyra nosyl phenyl sulfoxide as a glycosyl donor. Synthesis of some oligosaccharides containing an alpha-D-galactopyranosyl group", CARBOHYDRATE RESEARCH, PERGAMON, GB, vol. 233, 2 September 1992 (1992-09-02), pages 245-250, XP002223211, ISSN: 0008-6215, DOI: 10.1016/S0008-6215(00)90937-6
- Yukisirige Ito ET AL: "AN EFFICIENT APPROACH TO STEREOSELECTIVE GLYCOSYLATION OF N-ACETYLNEURAMINIC ACID: USE OF PHENYLSELENYL GROUP AS A STEREOCONTROLLING AUXILIARY", Tetrahedron Letters, 1 January 1987 (1987-01-01), pages 6221-6224, XP055238530, Retrieved from the Internet: URL:http://www.sciencedirect.com/science/a rticle/pii/S004040390061852X/pdf?md5=dbdb1 6bcf86b17d1c0d7177d7d5c9674&pid=1-s2.0-S00 4040390061852X-main.pdf
- Subramaniam Sabesan ET AL: "The conformational properties of the gangliosides based on 'H and I3C nuclear magnetic resonance studies", , 1 January 1984 (1984-01-01), XP055238536, Retrieved from the Internet: URL:http://www.nrcresearchpress.com/doi/pd f/10.1139/v84-172
- YU L ED - MATTOUSSI HEDI ET AL: "AMORPHOUS PHARMACEUTICAL SOLIDS: PREPARATION, CHARACTERIZATION AND STABILIZATION", ADVANCED DRUG DELIVERY REVIEWS, ELSEVIER, AMSTERDAM, NL, vol. 48, no. 1, 16 May 2001 (2001-05-16), pages 27-42, XP009065056, ISSN: 0169-409X, DOI: 10.1016/S0169-409X(01)00098-9

## Description

### Technical Field

The present invention relates to crystals of an oligosaccharide useful, for example, as raw materials for or as intermediates of health foods, pharmaceutical compositions, cosmetics, etc. and a process for producing crystals of an oligosaccharide.

### Background Art

Oligosaccharides are useful, for example, as raw materials for or as intermediates of health foods, pharmaceutical compositions, cosmetics, etc. Therefore, there is a demand for oligosaccharides of good storage stability and of high purity which do not contain impurities, decomposition products or the like. Some reports have been made on methods for synthesis or fermentation of oligosaccharides [Chem. Rev., Vol. 100, p. 4465 (2000); Curr. Opin. in Drug Discovery & Develop., Vol. 3, p. 756 (2000); WO98/12343; WO99/40205]. In these methods, end products are usually obtained as powders (amorphous) by freeze-drying treatment, and obtaining them as crystals is considered to be difficult. The powders (amorphous) obtained by freeze-drying treatment are generally known to have a problem in respect of stability because of their hygroscopicity, deliquescence, etc., and thus need to be refrigerated or frozen when stored, transported, distributed, etc. Therefore, there exists a demand for crystals of an oligosaccharide capable of being stored at ordinary temperatures and a process for production thereof for a large supply of oligosaccharides on an industrial scale.

Kuhn et al. (Chemische Berichte,1956 vol. 89(11),pp 2513-23) discloses the crystallisation of Fucosyllactose and Imberty et al. (carb. Res. 181(1988) 41-55) the crystallisation of panose. Another known example of crystals of an oligosaccharide is crystals of Lewis X [Gal β1, 4 (Fucα1, 2) GlcNAc], and a process for production thereof is known [Glycobiology, Vol. 6, p. 537 (1996)]. However, the process takes an extremely long time as two years for crystallization, and thus is not suitable for large-scale synthesis or industrialization. Therefore, there exists a demand for a process for producing crystals of an oligosaccharide capable of crystallization in a short time.

### Disclosure of the Invention

An object of the present invention is to provide crystals of an oligosaccharide useful, for example, as materials for or as intermediates of health foods, pharmaceutical compositions, cosmetics, etc. and a process for producing crystals of an oligosaccharide which is suitable for large-scale synthesis or industrialization.

The present invention relates to the following (1) to (11).
(1) A process for producing crystals of an oligosaccharide which comprises adding an aqueous solution containing an oligosaccharide represented by general formula (I): 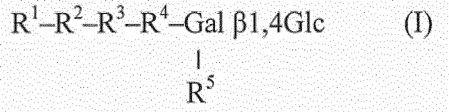 [wherein Gal represents galactose (hereinafter abbreviated in the same manner); Glc represents glucose (hereinafter abbreviated in the same manner); R¹ represents GlcNAc (wherein GlcNAc represents N-acetylglucosamine, which is hereinafter abbreviated in the same manner), NeuAc (wherein NeuAc represents N-acetylneuraminic acid, which is hereinafter abbreviated in the same manner), Gal, Fuc (wherein Fuc represents fucose, which is hereinafter abbreviated in the same manner) or GalNAc (wherein GalNAc represents N-acetylgalactosamine, which is hereinafter abbreviated in the same manner); R², R³ and R⁴, which may be the same or different, each represent a single bond, GlcNAc, NeuAc, Gal, Fuc or GalNAc; and R⁵ represents a hydrogen atom, GlcNAc, NeuAc, Gal, Fuc or GalNAc] to a water-miscible organic solvent, wherein the water-miscible organic solvent is methanol or acetone.
(2) The process for producing crystals of an oligosaccharide according to (1), wherein R², R³ and R⁴, which may be the same or different, each are GlcNAc, NeuAc, Gal, Fuc or GalNAc.
(3) The process for producing crystals of an oligosaccharide according to (1), wherein R⁴ is a single bond.
(4) The process for producing crystals of an oligosaccharide according to (1), wherein R³ and R⁴ each are a single bond.
(5) The process for producing crystals of an oligosaccharide according to (1), wherein R², R³ and R⁴ each are a single bond.
(6) The process for producing crystals of an oligosaccharide according to (3), wherein R¹ is GlcNAc; R² is Gal; R³ is GlcNAc; and R⁵ is a hydrogen atom.
(7) The process for producing crystals of an oligosaccharide according to (4), wherein R¹ is NeuAc or Gal; R² is GlcNAc or GalNAc; and R⁵ is a hydrogen atom.
(8) The process for producing crystals of an oligosaccharide according to (5), wherein R¹ is GlcNAc, Gal, NeuAc or Fuc; and R⁵ is a hydrogen atom or GalNAc.
(9) The process for producing crystals of an oligosaccharide according to (1), wherein at least one of R¹, R², R³, R⁴ and R⁵ is a deoxy sugar residue, and the aqueous solution containing an oligosaccharide is an aqueous solution obtained by treatment with a synthetic adsorption resin.
(10) The process for producing crystals of an oligosaccharide according to (9), wherein the deoxy sugar residue is Fuc.
(11) The process for producing crystals of an oligosaccharide according to (9), wherein R¹ is Fuc; R², R³ and R⁴ each are a single bond; and R⁵ is a hydrogen atom.

Described herein are the following (a) to (x).
(a) A process for producing crystals of an oligosaccharide comprising three or more monosaccharide residues which comprises adding an aqueous solution containing the oligosaccharide comprising three or more monosaccharide residues to a water-miscible organic solvent.
(b) A process for producing crystals of an oligosaccharide which comprises adding an aqueous solution containing an oligosaccharide represented by general formula (I): 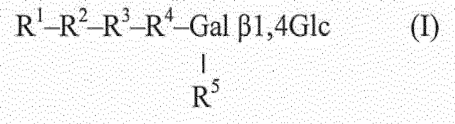 [wherein Gal represents galactose (hereinafter abbreviated in the same manner); Glc represents glucose (hereinafter abbreviated in the same manner); R¹ represents a monosaccharide residue, an amino sugar
   residue, or a derivative of the monosaccharide residue or the amino sugar residue; R², R³ and R⁴, which may be the same or different, each represent a monosaccharide residue, an amino sugar residue, a derivative of the monosaccharide residue or the amino sugar residue, -X(-Y)- (wherein X and Y, which may be the same or different, each represent a monosaccharide residue, an amino sugar residue, or a derivative of the monosaccharide residue or the amino sugar residue) or a single bond; and R⁵ represents a hydrogen atom, a monosaccharide residue, an amino sugar residue, or a derivative of the monosaccharide residue or the amino sugar residue] to a water-miscible organic solvent.
(c) The process for producing crystals of an oligosaccharide according to the above (b), wherein R¹ is GlcNAc (GlcNAc represents N-acetylglucosamine, which is hereinafter abbreviated in the same manner), NeuAc (NeuAc represents N-acetylneuraminic acid; which is hereinafter abbreviated in the same manner), Gal, Fuc (Fuc represents fucose, which is hereinafter abbreviated in the same manner) or GalNAc (GalNAc represents N-acetylgalactosamine, which is hereinafter abbreviated in the same manner); R², R³ and R⁴, which may be the same or different, each are a single bond, GlcNAc, NeuAc, Gal, Fuc or GalNAc; and R⁵ is a hydrogen atom, GlcNAc, NeuAc, Gal, Fuc or GalNAc.
(d) The process for producing crystals of an oligosaccharide according to the above (b) or (c), wherein R², R³ and R⁴, which may be the same or different, each are GlcNAc, NeuAc, Gal, Fuc or GalNAc.
(e) The process for producing crystals of an oligosaccharide according to the above (b) or (c), wherein R⁴ is a single bond.
(f) The process for producing crystals of an oligosaccharide according to the above (b) or (c), wherein R³ and R⁴ each are a single bond.
(g) The process for producing crystals of an oligosaccharide according to the above (b) or (c), wherein R², R³ and R⁴ each are a single bond.
(h) The process for producing crystals of an oligosaccharide according to the above (e), wherein R¹ is GlcNAc; R² is Gal; R³ is GlcNAc; and R⁵ is a hydrogen atom.
(i) The process for producing crystals of an oligosaccharide according to the above (f), wherein R¹ is NeuAc or Gal; R² is GlcNAc or GalNAc; and R⁵ is a hydrogen atom.
(j) The process for producing crystals of an oligosaccharide according to the above (i), wherein R¹ is GlcNAc, Gal, NeuAc or Fuc; and R⁵ is a hydrogen atom or GalNAc.
(k) The process for producing crystals of an oligosaccharide according to the above (b), wherein at least one of R¹, R², R³, R⁴ and R⁵ is a deoxy sugar residue, and the aqueous solution containing an oligosaccharide is an aqueous solution obtained by treatment with a synthetic adsorption resin.
(l) The process for producing crystals of an oligosaccharide according to the above (k), wherein the deoxy sugar residue is Fuc.
(m) The process for producing crystals of an oligosaccharide according to the above (k), wherein R¹ is Fuc; R², R³ and R⁴ each are a single bond; and R⁵ is a hydrogen atom.
(n) The process for producing crystals of an oligosaccharide according to any one of the above (a) to (m), wherein the water-miscible organic solvent is an alcohol or a ketone.
(o) The process for producing crystals of an oligosaccharide according to any one of the above (a) to (m), wherein the water-miscible organic solvent is methanol or acetone.
(p) A Crystal of an oligosaccharide represented by general formula (I) according to the above (b).
(q) The crystal of an oligosaccharide according to the above (p), wherein R¹ is GlcNAc, NeuAc, Gal, Fuc or GalNAc; R², R³ and R⁴, which may be the same or different, each are a single bond, GlcNAc, NeuAc, Gal, Fuc or GalNAc; and R⁵ is a hydrogen atom, GlcNAc, NeuAc, Gal, Fuc or GalNAc.
(r) The crystal of an oligosaccharide according to the above (p) or (q), wherein R², R³ and R⁴, which may be the same or different, each are GlcNAc, NeuAc, Gal, Fuc or GalNAc.
(s) The crystal of an oligosaccharide according to the above (p) or (q), wherein R⁴ is a single bond.
(t) The crystal of an oligosaccharide according to the above (p) or (q), wherein R³ and R⁴ each are a single bond.
(u) The crystal of an oligosaccharide according to the above (p) or (q), wherein R², R³ and R⁴ each are a single bond.
(v) The crystal of an oligosaccharide according to the above (s), wherein R¹ is GlcNAc; R² is Gal; R³ is GlcNAc; and R⁵ is a hydrogen atom.
(w) The crystal of an oligosaccharide according to the above (t), wherein R¹ is NeuAc or Gal; R² is GlcNAc or GalNAc; and R⁵ is a hydrogen atom.
(x) The crystal of an oligosaccharide according to the above (u), wherein R¹ is GlcNAc, Gal, NeuAc or Fuc; and R⁵ is a hydrogen atom or GalNAc.

Hereinafter, the oligosaccharides comprising three or more monosaccharide residues or the oligosaccharides represented by general formula (I) are referred to as Oligosaccharides (I), and the crystals of an Oligosaccharide (I) are referred to as Oligosaccharide Crystals (I).

The definitions of the groups in general formula (I) are explained below.
(i) The monosaccharide of the monosaccharide residue includes Gal, Glc, allose (All), arabinose (Ara), altrose (Alt), gulose (Gul), mannose (Man), talose (Tal), fructose (Fru), ribose (Rib), xylose (Xyl) and the like.
(ii) The amino sugar of the amino sugar residue includes neuraminic acid (Neu), muramic acid (Mur), glucosamine (GlcN), mannosamine (ManN), galactosamine (GalN), 2-amino-2-deoxyglucopyranose (GlcpN) and the like.
(iii) The derivatives of the monosaccharide residue or the amino sugar residue include uronic acids; deoxy sugars; derivatives wherein two members selected from the group consisting of monosaccharide residues [the monosaccharide residue has the same significance as the above monosaccharide residue (i)], amino sugar residues [the amino sugar residue has the same significance as the above amino sugar residue (ii)] and derivatives of the monosaccharide residue or the amino sugar residue (the derivatives of the amino sugar residue and the amino sugar residue include deoxy sugars etc.), which are the same or different, are linked by a glycoside bond; and derivatives wherein a hydroxyl group or an amino group in those is protected with acetyl or the like.
   Examples of the uronic acids are glucuronic acid (GlcA) and galacturonic acid (GalA); examples of the deoxy sugars are Fuc and rhamnose (Rha); and examples of the derivatives wherein a hydroxyl group, or an amino group in those is protected with acetyl or the like are GlcNAc, NeuAc, GalNAc and N-acetylmannosamine (ManNAc).
(iv) The glycoside bonds between R¹ and R², R² and R³, R³ and R⁴, R⁴ and Gal, and R⁵ and Gal (R¹, R², R³, R⁴ and R⁵ have the same significances as defined above, respectively) may be the same or different, and examples of the bonds include an α-1,2 bond, an α-2,3 bond, an α-1,4 bond, a β-1,3 bond and a β-1,4 bond. Examples of Oligosaccharides (I) formed by specifically preferred glycoside bonds include trisaccharides such as GlcNAcβ1, 3Galβ1, 4Glc, Galα1, 4Galβ1, 4Glc, NeuAcα2, 3Galβ1, 4Glc and Fucα1, 2Gal-β1, 4Glc, tetrasaccharides such as Galβ1, 4GlcNAcβ1, 3Gal-β1, 4Glc and NeuAcα2, 3(GalNAcβ1,4)Galβ1, 4Glc, and pentasaccharides such as GlcNAcβ1, 3Galβ1,4GlcNAcβ1, 3Gal-β1, 4Glc.
   The present disclosure is further described below.
(v) The oligosaccharide comprising three or more monosaccharide residues includes branched or straight-chain oligosaccharides wherein 3 to 20 members, preferably 3 to 10 members, more preferably 3 to 6 members selected from the group consisting of monosaccharide residues [the monosaccharide residue has the same significance as the above monosaccharide residue (i)], amino sugar residues [the amino sugar residue has the same significance as the above amino sugar residue (ii)] and derivatives of the monosaccharide residue or the amino sugar residue [the derivative of the monosaccharide residue or the amino sugar residue has the same significance as the above derivative of the monosaccharide residue or the amino sugar residue (iii)], which are the same or different, are linked with one another by glycoside bonds which may be the same or different (examples of the glycoside bonds are an α-1,2 bond, an α-2,3 bond, an α-1,4 bond, a β-1,3 bond and a β-1,4 bond).
(vi) The aqueous solution containing the oligosaccharide may be any aqueous solution that contains the oligosaccharide, but the saccharide purity of the oligosaccharide is preferably 50% or more, more preferably 70% or more. The aqueous solution may also comprise an organic solvent such as an alcohol (e.g., methanol, ethanol or isopropyl alcohol) or a ketone (e.g., acetone or methyl ethyl ketone). The water content of the aqueous solution is preferably 20% or more. Specific examples of the aqueous solution are those prepared by subjecting an oligosaccharide solution (e.g., a reaction solution, a culture medium or a cell-free culture medium obtained by synthesis or fermentation) to pretreatment (e.g., treatment with a membrane, gel filtration, treatment with activated carbon, treatment with an ion exchange resin, treatment with a synthetic adsorption resin or solvent precipitation). Preferred pretreatments are treatment with activated carbon, treatment with an ion exchange resin, treatment with a synthetic adsorption resin and solvent precipitation, among which solvent precipitation and treatment with a synthetic adsorption resin are particularly preferred. These treatments may be appropriately employed in combination. In particular, treatment with a synthetic adsorption resin is preferred as the pretreatment to obtain an aqueous solution containing Oligosaccharide (I) in which at least one of the monosaccharide residues is Fuc.
(vii) The water-miscible organic solvent includes any organic solvents that are miscible with water. Preferred are alcohols such as methanol, ethanol and isopropyl alcohol, and ketones such as acetone and methyl ethyl ketone.
(viii) The synthetic adsorption resin includes nonpolar and porous adsorption resins such as DIAION HP resins (e.g., HP10, HP20, HP21, HP30, HP40 and HP50; Mitsubishi Chemical Corporation), DIAION SP800 resins (e.g., SP800, SP825, SP850 and SP875; Mitsubishi Chemical Corporation), DIAION SP200 resins (e.g., SP205, SP206, SP207 and SP207SS; Mitsubishi Chemical Corporation) and Amberlite XAD resins (e.g., XAD4, XAD7HP, XAD16 and XAD1600; Rohm and Haas).
(ix) The crystal(s) of an oligosaccharide may be of any crystalline form, for example, columns, plates or needles. Particularly preferred are columns.

The process for producing Oligosaccharide Crystals (I) is described in detail below.

### Production process:

A reaction solution, a culture medium or a cell-free culture medium containing Oligosaccharide (I) obtained by a synthesis method or a fermentation method is pretreated according a known method [e.g., Chem. Rev., Vol. 100, p. 4465 (2000); Curr. Opin. in Drug Discovery & Develop, Vol. 3, p. 756 (2000); WO98/12343; and WO99/40205] to prepare an aqueous solution containing Oligosaccharide (I) whose saccharide purity is 50% or more, preferably 70% or more. The obtained solution containing Oligosaccharide (I) is added dropwise to a water-miscible organic solvent which is a bad solvent at a temperature between -20°C and the boiling point of the water-miscible organic solvent or under reflux for one minute to 10 hours, preferably 10 minutes to 2 hours. After the completion of dropping, the resulting mixture is stirred at a temperature between -20°C and the boiling point of the water-miscible organic solvent or under reflux for 1 to 20 hours, preferably 2 to 4 hours to deposit crystals. The deposited crystals are separated by centrifugal filtration, decantation or the like, washed with water or a water-miscible organic solvent, and then dried under reduced pressure or by airflow to obtain Oligosaccharide Crystals (I). Oligosaccharide Crystals (I) can be further purified by carrying out operations such as washing, drying and recrystallization.

The pretreatments to obtain the aqueous solution containing Oligosaccharide (I) include treatment with a membrane, gel filtration, treatment with activated carbon, treatment with an ion exchange resin, treatment with a synthetic adsorption resin and solvent precipitation. Preferred are treatment with activated carbon, treatment with an ion exchange resin, treatment with a synthetic adsorption resin and solvent precipitation, among which solvent precipitation and treatment with a synthetic adsorption resin are particularly preferred. These treatments may be appropriately employed in combination. In particular, treatment with a synthetic adsorption resin is preferred as the pretreatment to obtain an aqueous solution containing Oligosaccharide (I) wherein at least one of the monosaccharide residues is Fuc.

The water-miscible organic solvent can be used alone, or as a mixture of two or more kinds or a mixture with water.

In addition to the above-described process, Oligosaccharide Crystals (I) can also be obtained by general crystallization methods such as a method in which the aqueous solution containing Oligosaccharide (I) is concentrated, cooled and neutralized, and a method in which a water-miscible organic solvent as a bad solvent is added to the aqueous solution containing Oligosaccharide (I) to promote the formation of Oligosaccharide Crystals (I).

Oligosaccharide Crystals (I) obtained by the above processes may be obtained as adducts with water or with various water-miscible organic solvents.

Oligosaccharide Crystals (I) obtained by the above processes sometimes exist in different crystalline forms or different grain sizes, and these can be obtained alone or as a mixture.

Specific examples of Oligosaccharide Crystals (I) obtained by the above processes are shown in Table 1.

**Table 1**

| Example No. | Crystals No. | Oligosaccharide Crystals |
|---|---|---|
| 1 | 1 | GlcNAcβ1, 3Galβ1, 4Glc |
| 2 | 2 | Galβ1, 4GlcNAcβ1, 3Galβ1, 4Glc |
| 3 | 3 | GlcNAcβ1, 3Galβ1, 4GlcNAcβ1, 3Galβ1, 4Glc |

The storage stability of Oligosaccharide Crystals (I) of the present invention is illustrated in the following test example.

### Test Example: Comparison of Stability of Oligosaccharide Crystals (I) and freeze-dried Oligosaccharide (I) powders

The storage stability of Oligosaccharide Crystals (I) obtained in Examples 1 to 3 and freeze-dried Oligosaccharide (I) powders obtained in Reference Examples 1 to 3 was examined by keeping them at 105°C in the atmosphere under ordinary pressure for 20 days and measuring the residual rate of Oligosaccharides (I). The results are shown in Table 2.

The residual rate of Oligosaccharides (I) in the samples was measured by high performance liquid chromatography (HPLC) and expressed in terms of HPLC purity (%).

Measurement conditions for HPLC are as follows.
Analyzer: product of Dionex Corporation
Column: CarboPac PA 10
Column temperature: 30°C
Mobile phase: 10-100% aqueous solution of NaOH (500 mmol/L) (Gradient elution in 11 minutes)
Flow rate: 0.8 mL/minute
Detection: electrochemical detection (PAD method)

**Table 2**

| | Residual rate of oligosaccharides: HPLC purity (%) | | | | |
|---|---|---|---|---|---|
| | Days that passed | | | | |
| | 0 | 1 | 4 | 7 | 20 |
| GlcNAcβ1, 3Galβ1, 4Glc Crystals (Crystals No. 1) | 98.0 | 99.7 | 99.7 | 99.7 | 98.9 |
| Freeze-dried GlcNAcβ1, 3Galβ1, 4Glc powders | 99.8 | 99.7 | 97.0 | 98.8 | 93.8 |
| Galβ1, 4GlcNAcβ1, 3Gal-β1, 4Glc Crystals (Crystals No. 2) | 99.3 | 99.0 | 99.2 | 99.0 | 98.8 |
| Freeze-dried Galβ1, AGlcNAc-β1, 3Galβ1, 4Glc powders | 99.3 | 98.6 | 97.6 | 96.2 | 90.9 |
| GlcNAcβ1, 3Galβ1, 4GlcNAcβ1, 3Galβ1, 4Glc Crystals (Crystals No. 3) | 98.8 | 99.3 | 99.0 | 99.0 | 99.3 |
| Freeze-dried GlcNAcβ1, 3Gal-β1, 4GlcNAcβ1, 3Galβ1, 4Glc powders | 98.6 | 98.2 | 97.4 | 95.4 | 85.2 |

As is clear from Table 2, HPLC analysis revealed a fall in the residual rate of Oligosaccharides (I) and remarkable decomposition of the freeze-dried Oligosaccharide (1) powders obtained in reference examples. On the contrary, there was observed no fall in the residual rate of Oligosaccharide (I) in Oligosaccharide Crystals (I) obtained by the process of the present invention. It indicates that Oligosaccharide Crystals (I) are extremely stable.

### Best Modes for Carrying Out the Invention

Certain embodiments of the present invention are illustrated in detail in the following examples and reference examples. These examples and reference examples are not to be construed as limiting the scope of the present invention.

### Example 1: Production of GlcNAcβ1, 3Galβ1, 4Glc Crystals

The GlcNAcβ1, 3Galβ1, 4Glc reaction solution obtained in Reference Example 4 was centrifuged to remove cells and passed through a column of DIAION SK-1B (H type, Mitsubishi Chemical Corporation) and then a column of DIAION WA-30 (OH type, Mitsubishi Chemical Corporation) for desalting. The resulting solution was adjusted to pH 6.5 with HCl and concentrated under reduced pressure to obtain a treated solution of GlcNAcβ1, 3Galβ1, 4Glc (aqueous solution: 100 mL, 200 g/L). The obtained solution was gradually added to methanol heated to 60°C (500 mL) in about 30 minutes, and the resulting mixture was refluxed at 60°C for about 3 hours for crystallization. The resulting mixture was cooled to 20°C and stirred for one hour. Then, crystals were separated by filtration and washed with methanol. The obtained crystals were dried by airflow, whereby 14 g of GlcNAcβ1, 3Galβ1, 4Glc Crystals was obtained.

Powder X-ray diffraction data of the crystals are shown in Table 3.

**Table 3**

| Powder X-ray diffraction data of GlcNAcβ1, 3Galβ1, 4Glc Crystals | | | |
|---|---|---|---|
| d (A) | I/I₀ (%) | d (A) | I/I₀ (%) |
| 10.773 | 41 | 4.027 | 42 |
| 9.253 | 74 | 3.855 | 28 |
| 6.992 | 36 | 3.774 | 36 |
| 5.336 | 22 | 3.697 | 24 |
| 4.779 | 100 | 3.558 | 21 |
| 4.618 | 46 | 3.311 | 54 |
| 4.537 | 49 | 3.030 | 33 |
| 4.491 | 98 | 2.811 | 19 |
| 4.236 | 59 | 2.630 | 22 |
| 4.129 | 65 | | |

### Example 2: Production of Galβ1, 4GlcNAcβ1, 3Galβ1, 4Glc Crystals

The Galβ1,4 GlcNAcβ1,3Galβ1,4Glc reaction solution obtained in Reference Example 5 was centrifuged to remove cells and passed through a column of DIAION SK-1B (H type, Mitsubishi Chemical Corporation) and then a column of DIAION WA-30 (OH type, Mitsubishi Chemical Corporation) for desalting. The resulting solution was adjusted to pH 6.5 with HCl and concentrated under reduced pressure to obtain a treated solution of Galβ1,4GlcNAcβ1,3Galβ1,4Glc (aqueous solution: 70 mL, 300 g/L). The obtained solution was gradually added to acetone heated to 58°C (500 mL) in about 30 minutes, and the resulting mixture was refluxed at 58°C for about 2 hours for crystallization. The resulting mixture was cooled to 20°C and stirred for one hour. Then, crystals were separated by filtration and washed with acetone. The obtained crystals were dried by airflow, whereby 16 g of Galβ1,4GlcNAcβ1,3Galβ1,4Glc Crystals was obtained.

Powder X-ray diffraction data of the crystals are shown in Table 4.

**Table 4**

| Powder X-ray diffraction data of Galβ1, 4GlcNAcβ1, 3Galβ1, 4Glc Crystals | | | |
|---|---|---|---|
| d (A) | I/I₀ (%) | d (A) | I/I₀ (%) |
| 18.588 | 15 | 4.560 | 100 |
| 13.281 | 22 | 4.381 | 98 |
| 11.182 | 30 | 4.277 | 52 |
| 10.644 | 34 | 4.055 | 34 |
| 9.253 | 16 | 3.888 | 27 |
| 8.147 | 10 | 3.580 | 23 |
| 7.824 | 11 | 3.299 | 19 |
| 6.804 | 20 | 3.235 | 20 |
| 6.167 | 12 | 2.708 | 15 |
| 5.843 | 14 | 2.453 | 14 |
| 5.639 | 21 | 2.354 | 18 |
| 4.897 | 18 | 2.321 | 18 |
| 4.679 | 66 | | |

### Example 3: Production of GlcNAcβ1, 3Galβ1, 4GlcNAcβ1, 3Galβ1, 4Glc Crystals

The GlcNAcβ1, 3Galβ1, 4GlcNAcβ1, 3Galβ1, 4Glc reaction solution obtained in Reference Example 6 was centrifuged to remove cells and passed through a column of DIAION SK-1B (H type, Mitsubishi Chemical Corporation) and then a column of DIAION WA-30 (OH type, Mitsubishi Chemical Corporation) for desalting. The resulting solution was adjusted to pH 6.5 with HCl and concentrated under reduced pressure to obtain a treated solution of GlcNAcβ1, 3Galβ1, 4GlcNAcβ1, 3Galβ1, 4Glc (aqueous solution: 100 mL, 200 g/L). The obtained solution was gradually added to methanol heated to 60°C (500 mL) in about 30 minutes, and the resulting mixture was refluxed at 60°C for about 3 hours for crystallization. The resulting mixture was cooled to 20°C and stirred for one hour. Then, crystals were separated by filtration and washed with methanol. The obtained crystals were dried by airflow, whereby 16 g of GlcNAcβ1, 3Galβ1, 4GlcNAcβ1, 3Galβ1, 4Glc Crystals was obtained.

Powder X-ray diffraction data of the crystals are shown in Table 5.

**Table 5**

| Powder X-ray diffraction data of GlcNAcβ1, 3Galβ1, 4GlcNAcβ1, 3Galβ1, 4Glc Crystals | | | |
|---|---|---|---|
| d (A) | I/I₀ (%) | d (A) | I/I₀ (%) |
| 20.5322 | 8 | 3.5037 | 16 |
| 12.0996 | 16 | 3.4242 | 20 |
| 11.2531 | 15 | 3.3607 | 16 |
| 10.7084 | 17 | 3.2936 | 13 |
| 9.4509 | 40 | 3.1729 | 18 |
| 7.1037 | 8 | 3.1026 | 9 |
| 6.3887 | 7 | 3.0305 | 10 |
| 5.7863 | 9 | 2.8915 | 7 |
| 5.3042 | 9 | 2.8466 | 9 |
| 5.0350 | 8 | 2.8074 | 10 |
| 4.6189 | 100 | 2.6728 | 11 |
| 4.3710 | 28 | 2.6384 | 9 |
| 4.2874 | 32 | 2.5687 | 9 |
| 4.0920 | 26 | 2.5232 | 8 |
| 3.9397 | 20 | 2.4728 | 10 |
| 3.7985 | 18 | 2.3600 | 11 |
| 3.6672 | 14 | 2.3335 | 10 |
| 3.5729 | 13 | | |

### Reference Example 1: Production of freeze-dried GlcNAcβ1, 3Galβ1, 4Glc powders

The GlcNAcβ1, 3Galβ1, 4Glc Crystals obtained in Example 1 (5 g) were dissolved in water to obtain a GlcNAcβ1,3Galβ1,4Glc solution (10 mL, 500 g/L). The solution was frozen at -30°C and then dried in a freeze-dryer to obtain 4.8 g of freeze-dried GlcNAcβ1, 3Galβ1, 4Glc powders.

### Reference Example 2: Production of freeze-dried Galβ1, 4GlcNAcβ1, 3Galβ1, 4Glc powders

The Galβ1, 4GlcNAcβ1, 3Galβ1, 4Glc Crystals obtained in Example 2 (5 g) were dissolved in water to obtain a Galβ1, 4GlcNAcβ1, 3Galβ1, 4Glc solution (10 mL, 500 g/L). The solution was frozen at -30°C and then dried in a freeze-dryer to obtain 4.5 g of freeze-dried Galβ1, 4GlcNAcβ1, 3Galβ1, 4Glc powders.

### Reference Example 3: Production of freeze-dried GlcNAcβ1, 3Galβ1, 4GlcNAcβ1, 3Galβ1, 4Glc powders

The GlcNAcβ1, 3Galβ1, 4GlcNAcβ1, 3Galβ1, 4Glc Crystals obtained in Example 3 (5 g) were dissolved in water to obtain a GlcNAcβ1, 3Galβ1, 4GlcNAcβ1, 3Galβ1, 4Glc solution (10 ml, 500 g/l). The solution was frozen at -30°C and then dried in a freeze-dryer to obtain 4.7 g of freeze-dried GlcNAcβ1, 3Galβ1, 4GlcNAcβ1, 3Galβ1, 4Glc powders.

### Reference Example 4: GlcNAcβ1, 3Galβ1, 4Glc reaction solution

A GlcNAcβ1, 3Galβ1, 4Glc reaction solution was obtained from uridine diphosphate-N-acetylglucosamine obtained by the method described in WO98/12343 and lactose using recombinant Escherichia coli highly expressing the enzyme described in Glycobiology, Vol. 9, p. 1061 (1999) according to the method for producing sugar chains described in WO98/12343.

### Reference Example 5: Galβ1, 4GlcNAcβ1, 3Galβ1, 4Glc reaction solution

A Galβ1, 4GlcNAcβ1, 3Galβ1, 4Glc reaction solution was obtained using the GlcNAcβ1, 3Galβ1, 4Glc reaction solution obtained in Reference Example 4 and uridine diphosphate-galactose according to the method described in Reference Example 4.

### Reference Example 6: GlcNAcβ1, 3Galβ1, 4GlcNAcβ2, 3Galβ1, 4Glc reaction solution

A GlcNAcβ1, 3Galβ1, 4GlcNAcβ1, 3Galβ1, 4Glc reaction solution is obtained from uridine diphosphate-N-acetylglucosamine obtained by the method described in WO98/12343 and the Galβ1, 4GlcNAcβ1, 3Galβ1, 4Glc reaction solution obtained in Reference Example 5 according to the method described in Reference Example 4.

### Industrial Applicability

The present invention provides crystals of an oligosaccharide useful, for example, as raw materials for or as intermediates of health foods, pharmaceutical compositions, cosmetics, etc. and a process for producing crystals of an oligosaccharide which is suitable for large-scale synthesis or industrialization.

## Claims

1. A process for producing crystals of an oligosaccharide which comprises adding an aqueous solution containing an oligosaccharide represented by general formula (I): 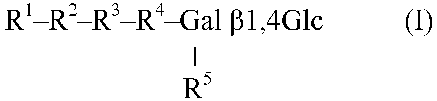 [wherein Gal represents galactose (hereinafter abbreviated in the same manner); Glc represents glucose (hereinafter abbreviated in the same manner); R¹ represents GlcNAc (wherein GlcNAc represents N-acetylglucosamine, which is hereinafter abbreviated in the same manner), NeuAc (wherein NeuAc represents N-acetylneuraminic acid, which is hereinafter abbreviated in the same manner), Gal, Fuc (wherein Fuc represents fucose, which is hereinafter abbreviated in the same manner) or GalNAc (wherein GalNAc represents N-acetylgalactosamine, which is hereinafter abbreviated in the same manner) ; R², R³ and R⁴, which may be the same or different, each represent a single bond, GlcNAc, NeuAc, Gal, Fuc or GalNAc; and R⁵ represents a hydrogen atom, GlcNAc, NeuAc, Gal, Fuc or GalNAc] to a water-miscible organic solvent, wherein the water-miscible organic solvent is methanol or acetone.

2. The process for producing crystals of an oligosaccharide according to Claim 1, wherein R², R³ and R⁴, which may be the same or different, each are GlcNAc, NeuAc, Gal, Fuc or GalNAc.

3. The process for producing crystals of an oligosaccharide according to Claim 1, wherein R⁴ is a single bond.

4. The process for producing crystals of an oligosaccharide according to Claim 1, wherein R³ and R⁴ each are a single bond.

5. The process for producing crystals of an oligosaccharide according to Claim 1, wherein R², R³ and R⁴ each are a single bond.

6. The process for producing crystals of an oligosaccharide according to Claim 3, wherein R¹ is GlcNAc; R² is Gal; R³ is GlcNAc; and R⁵ is a hydrogen atom.

7. The process for producing crystals of an oligosaccharide according to Claim 4, wherein R¹ is NeuAc or Gal; R² is GlcNAc or GalNAc; and R⁵ is a hydrogen atom.

8. The process for producing crystals of an oligosaccharide according to Claim 5, wherein R¹ is GlcNAc, Gal, NeuAc or Fuc; and R⁵ is a hydrogen atom or GalNAc.

9. The process for producing crystals of an oligosaccharide according to Claim 1, wherein at least one of R¹, R², R³, R⁴ and R⁵ is a deoxy sugar residue, and the aqueous solution containing an oligosaccharide is an aqueous solution obtained by treatment with a synthetic adsorption resin.

10. The process for producing crystals of an oligosaccharide according to Claim 9, wherein the deoxy sugar residue is Fuc.

11. The process for producing crystals of an oligosaccharide according to Claim 9, wherein R¹ is Fuc; R², R³ and R⁴ each are a single bond; and R⁵ is a hydrogen atom.

## Patentansprüche

1. Verfahren zur Herstellung von Kristallen eines Oligosaccharids, umfassend Zugeben einer wässrigen Lösung, die ein durch die allgemeine Formel (I) dargestelltes Oligosaccharid enthält, 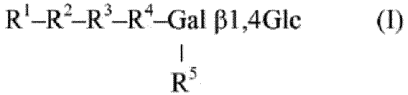 [wobei Gal Galactose darstellt (nachstehend auf die gleiche Weise abgekürzt); Glc Glucose darstellt (nachstehend auf die gleiche Weise abgekürzt); R¹ GlcNAc (wobei GlcNAc N-Acetylglucosamin darstellt, das nachstehend auf die gleiche Weise abgekürzt wird), NeuAc (wobei NeuAc N-Acetylneuraminsäure darstellt, die nachstehend auf die gleiche Weise abgekürzt wird), Gal, Fuc (wobei Fuc Fucose darstellt, die nachstehend auf die gleiche Weise abgekürzt wird) oder GalNac (wobei GalNac N-Acetylgalactosamin darstellt, das nachstehend auf die gleiche Weise abgekürzt wird) darstellt; R², R³ und R⁴, die gleich oder verschieden sein können, jeweils eine Einfachbindung, GlcNAc, NeuAc, Gal, Fuc oder GalNAc darstellen; und R⁵ ein Wasserstoffatom, GlcNAc, NeuAc, Gal, Fuc oder GalNAc darstellt] zu einem wassermischbaren organischen Lösungsmittel, wobei das wassermischbare organische Lösungsmittel Methanol oder Aceton ist.

2. Verfahren zur Herstellung von Kristallen eines Oligosaccharids gemäß Anspruch 1, wobei R², R³ und R⁴, die gleich oder verschieden sein können, jeweils GlcNAc, NeuAc, Gal, Fuc oder GalNAc sind.

3. Verfahren zur Herstellung von Kristallen eines Oligosaccharids gemäß Anspruch 1, wobei R⁴ eine Einfachbindung ist.

4. Verfahren zur Herstellung von Kristallen eines Oligosaccharids gemäß Anspruch 1, wobei R³ und R⁴ jeweils eine Einfachbindung sind.

5. Verfahren zur Herstellung von Kristallen eines Oligosaccharids gemäß Anspruch 1, wobei R², R³ und R⁴ jeweils eine Einfachbindung sind.

6. Verfahren zur Herstellung von Kristallen eines Oligosaccharids gemäß Anspruch 3, wobei R¹ GlcNAc ist; R² Gal ist; R³ GlcNAc ist; und R⁵ ein Wasserstoffatom ist.

7. Verfahren zur Herstellung von Kristallen eines Oligosaccharids gemäß Anspruch 4, wobei R¹ NeuAc oder Gal ist; R² GlcNAc oder GalNAc ist; und R⁵ ein Wasserstoffatom ist.

8. Verfahren zur Herstellung von Kristallen eines Oligosaccharids gemäß Anspruch 5, wobei R¹ GlcNAc, Gal, NeuAc oder Fuc ist; und R⁵ ein Wasserstoffatom oder GalNAc ist.

9. Verfahren zur Herstellung von Kristallen eines Oligosaccharids gemäß Anspruch 1, wobei wenigstens eines von R¹, R², R³, R⁴ und R⁵ ein Desoxy-Zuckerrest ist und die wässrige Lösung, die ein Oligosaccharid enthält, eine wässrige Lösung ist, die durch Behandlung mit einem synthetischen Adsorptionsharz erhalten ist.

10. Verfahren zur Herstellung von Kristallen eines Oligosaccharids gemäß Anspruch 9, wobei der Desoxy-Zuckerrest Fuc ist.

11. Verfahren zur Herstellung von Kristallen eines Oligosaccharids gemäß Anspruch 9, wobei R¹ Fuc ist; R², R³ und R⁴ jeweils eine Einfachbindung sind; und R⁵ ein Wasserstoffatom ist.

## Revendications

1. Procédé pour la production de cristaux d'un oligosaccharide qui comprend l'ajout d'une solution aqueuse contenant un oligosaccharide représenté par la formule générale (I) : 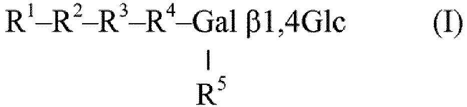 [dans lequel Gal représente le galactose (ci-après désigné par la même abréviation) ; Glc représente le glucose (ci-après désigné par la même abréviation) ; R¹ représente GlcNAc (GlcNAc représentant la N-acétyl-glucosamine, qui est ci-après désignée par la même abréviation), NeuAc (NeuAc représentant l'acide *N*-acétylneuraminique, qui est ci-après désigné par la même abréviation), Gal, Fuc (Fuc représentant le fucose, qui est ci-après désigné par la même abréviation) ou GalNAc (GalNAc représentant la N-acétylgalactosamine, qui est ci-après désignée par la même abréviation) ; R², R³ et R⁴, qui peuvent être identiques ou différents, représentent chacun une liaison simple, GlcNAc, NeuAc, Gal, Fuc ou GalNAc ; et R⁵ représente un atome d'hydrogène, GlcNAc, NeuAc, Gal, Fuc ou GalNAc] à un solvant organique miscible avec l'eau, dans lequel le solvant organique miscible avec l'eau est le méthanol ou l'acétone.

2. Procédé pour la production de cristaux d'un oligosaccharide selon la revendication 1, dans lequel R², R³ et R⁴, qui peuvent être identiques ou différents, sont chacun GlcNAc, NeuAc, Gal, Fuc ou GalNAc.

3. Procédé pour la production de cristaux d'un oligosaccharide selon la revendication 1, dans lequel R⁴ est une liaison simple.

4. Procédé pour la production de cristaux d'un oligosaccharide selon la revendication 1, dans lequel R³ et R⁴ sont chacun une liaison simple.

5. Procédé pour la production de cristaux d'un oligosaccharide selon la revendication 1, dans lequel R², R³ et R⁴ sont chacun une liaison simple.

6. Procédé pour la production de cristaux d'un oligosaccharide selon la revendication 3, dans lequel R¹ est GlcNAc ; R² est Gal ; R³ est GlcNAc ; et R⁵ est un atome d'hydrogène.

7. Procédé pour la production de cristaux d'un oligosaccharide selon la revendication 4, dans lequel R¹ est NeuAc ou Gal ; R² est GlcNAc ou GalNAc ; et R⁵ est un atome d'hydrogène.

8. Procédé pour la production de cristaux d'un oligosaccharide selon la revendication 5, dans lequel R¹ est GlcNAc, Gal, NeuAc ou Fuc ; et R⁵ est un atome d'hydrogène ou GalNAc.

9. Procédé pour la production de cristaux d'un oligosaccharide selon la revendication 1, dans lequel au moins l'un de R¹, R², R³, R⁴ et R⁵ est un résidu de désoxyose et la solution aqueuse contenant un oligosaccharide est une solution aqueuse obtenue par traitement avec une résine d'adsorption synthétique.

10. Procédé pour la production de cristaux d'un oligosaccharide selon la revendication 9, dans lequel le résidu de désoxyose est Fuc.

11. Procédé pour la production de cristaux d'un oligosaccharide selon la revendication 9, dans lequel R¹ est Fuc ; R², R³ et R⁴ sont chacun une liaison simple ; et R⁵ est un atome d'hydrogène.
